(19) **Europäisches Patentamt / European Patent Office / Office européen des brevets**

(11) **EP 2 075 723 A2**

(12) **EUROPEAN PATENT APPLICATION**

(43) Date of publication:
**01.07.2009 Bulletin 2009/27**

(51) Int Cl.:
**G06F 19/00** (2006.01)

(21) Application number: **08022483.5**

(22) Date of filing: **29.12.2008**

| | |
|---|---|
| (84) Designated Contracting States:<br>**AT BE BG CH CY CZ DE DK EE ES FI FR GB GR HR HU IE IS IT LI LT LU LV MC MT NL NO PL PT RO SE SI SK TR**<br>Designated Extension States:<br>**AL BA MK RS** | (72) Inventors:<br>• **Kim, Hyoung Jin**<br>  **Seoul 135-280 (KR)**<br>• **Lee, Jae Keun**<br>  **Seoul 135-280 (KR)** |
| (30) Priority: **28.12.2007 KR 20070140970** | (74) Representative: **Raunecker, Klaus Peter**<br>**Lorenz & Kollegen**<br>**Patent- und Rechtsanwaltskanzlei**<br>**Alte Ulmer Straße 2**<br>**D-89522 Heidenheim (DE)** |
| (71) Applicant: **MEDISON CO., LTD.**<br>**Kangwon-do 250-870 (KR)** | |

(54) **Ultrasound system and method of forming ultrasound image signals with multiprocessing**

(57)    The present invention relates to an ultrasound system. The ultrasound system comprises, a probe for transmitting an ultrasound beam along a plurality of scan lines in a target object and output receive signals based on ultrasound echoes reflected from the target object; a beam forming unit for applying delays to the receive signals and sum the delayed receive signals to thereby form scan line signals corresponding to the respective scan lines; a multiprocessing unit including a plurality of processors, each being configured to individually process a part of the scan line signals to output sub-image signals, the multiprocessing unit being configured to synthesize the sub-image signals to output ultrasound image signals corresponding to one image frame; and an image processing unit for performing an image processing upon the ultrasound image signals for display.

## FIG. 1

## Description

The present application claims priority from Korean Patent Application No. 10-2007-0140970 filed on December 28, 2007, the entire subject matter of which is incorporated herein by reference.

## BACKGROUND OF THE INVENTION

[Technical Field]

The present invention generally relates to ultrasound systems, and more particularly to an ultrasound system and a method of forming ultrasound image signals with multiprocessing.

[Background Art]

An ultrasound system has become an important and popular diagnostic tool due to its non-invasive and non-destructive nature. Modem high-performance ultrasound imaging diagnostic devices and techniques are commonly used to produce two- or three-dimensional images of internal features of patients.

The ultrasound system may include a transducer for generating ultrasound signals in response to electrical pulse signals and converting ultrasound echoes reflected from a target object into electrical receive signals. The ultrasound system may further include separate image processing modules such as a beam forming application specific integrated circuit (ASIC), a signal processing ASIC, a digital signal processing chip and the like. The image processing modules may sequentially process the receive signals outputted from the transducer in a pipeline manner, thereby obtaining ultrasound image signals-However, the use of the separate hardware-based image processing modules is not economical Also, it may be difficult to miniaturize the ultrasound system as well as to maintain and manage the ultrasound system.

## BRIEF DESCRIPTIN OF THE DRAWINGS

FIG. 1 is a block diagram showing an illustrative embodiment of an ultrasound system.

FIG. 2 is a schematic diagram showing an example of dividing scan line signals by a signal allocating unit.

## DETAINED DESCRIPTION OF THE INVENTION

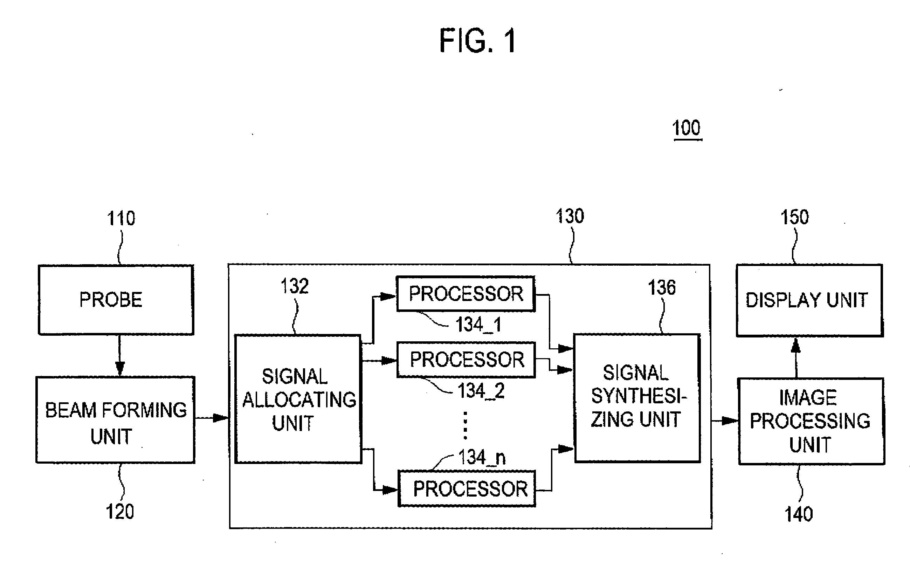

FIG. 1 is a block diagram showing an illustrative embodiment of an ultrasound system. The ultrasound system 100 may include a probe for transmitting and receiving ultrasound signals. The probe may include an array transducer comprising a plurality of elements The elements may be formed with a piezoelectric material adapted to reciprocally convert electrical signals and ultrasound signals. The elements may generate ultrasound signals in response to electrical pulse signals provided from pulsers (not shown). The ultrasound signals may be transmitted along a plurality of scan lines. Also, the transduce elements may convert ultrasound echoes reflected from a target object into electrical receive signals. The transducer elements may output the receive signals through channels.

The ultrasound system 100 may further include a beam forming unit 120, which may be configured to delay the receive signals inputted through the channels in consideration of distances between the elements and focal points. The beam forming unit 120 may be further configured to sum the delayed receive signals, thereby forming raw signals corresponding to the respective scan lines (hereinafter referred to as "scan line signals").

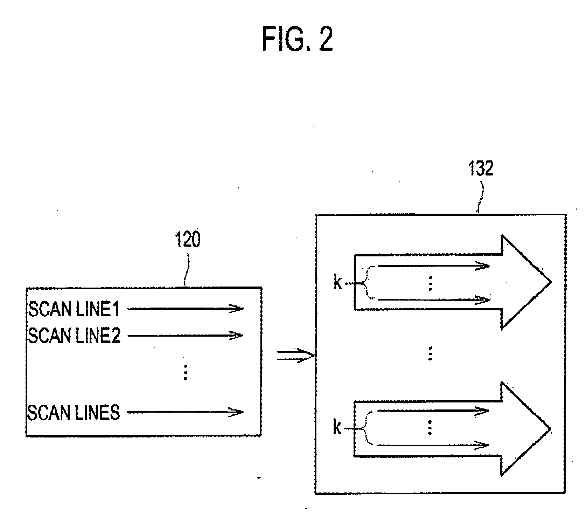

The ultrasound system 100 may further include a multiprocessing unit 130 configured to process the scan line signals in order to form ultrasound image signals. The multiprocessing unit 130 may include a signal allocating unit 132, a plurality of processors 134_1-134_n and a signal synthesizing unit 136. The signal allocating unit 132 may divide the scan line signals into a plurality of groups to allocate the scan line signals to the processors 134_1-134_n, as illustrated in FIG. 2. The number of groups may be determine depending on the number of processors 134_1-134_n. Assuming that the total number of scan lines is "S," the number of scan lines "k" associated with the scan line signals included in each of the groups may be computed by using the following equation (1).

$$S/n = k \qquad (1)$$

wherein "n" represents the number of processors 134_1-134_n.

Each of the processors 134_1-134_n may receive the scan line signals associate with the k scan lines allocated by the signal allocating unit 132. Each of the processors 134_1-134_n may be configured to process the scan line signals to form sub-image signals. The processors 134_1-134_n may amplify the scan line signals so as to have amplitudes suitable for image processing in the ultrasound system 100. The processors 134_1-134_n may be further configured to perform compensation for the attenuation losses in the amplified signals. Also, the processors 134_1-134_n may compress the amplified signals so as to have a dynamic range of a display unit and demodulate compressed signals into single pulse signals or spike signals. Further, the processors 134_1-134_n may be configured to perform a reject control for removing noise signals from the demodulated signals to thereby form the sub-image signals.

In one embodiment, although multiprocessing is achieved by the separate processors 134_1-134_n, the multiprocessing may be achieved by a multi core processor in anther embodiment of the present invention.

The signal synthesizing unit 136 may be configured to synthesize the sub-image signals outputted from the processors 134_1-134_n, thereby outputting the ultrasound image signals corresponding to an image frame. The multiprocessing unit 130 may further include a memory (not shown). The memory may store software algorithms necessary for processing the scan line signals.

The ultrasound system 100 may further include an image processing unit 140-The image processing unit 140 may be configured to perform image processing upon the ultrasound image signals for display. For example, the image processing unit 140 may scan-convert the ultrasound image signals into an image format suitable for display. The image processing unit 140 may be further configured to render the scan-converted ultrasound image signals to form pixel data. The ultrasound system 100 may also include a display unit 150 for displaying an ultrasound image on a screen based on the pixel data.

In one embodiment, although it is described that the scan conversion of the ultrasound image signals is carried out by the image processing unit 140 after synthesizing the sub-image signals, the scan conversion may be carried out in the multiprocessing unit 130 in another embodiment. That is, each of the processors 134_1-134_n may scan convert the sub-image signals and the signals synthesizing unit 136 may synthesize the sub-image signals, which are scan converted. In such a case, the image processing unit 140 may be further configured to tender the ultrasound image signals to form pixel data without performing the scan conversion.

The ultrasound system 100 may operate in a duplex mode for providing two or more different mode images, e.g., a B-mode image and a power Doppler mode image at the same time. Amounts and operations for processing the image data for the respective diagnostic modes may be different from each other. Thus, the processors 134_1-134_n may be divided into a plurality of processor groups depending on the number of diagnostic modes. Assuming that a B mode image and a power Doppler mode image are formed in the duplex mode, for example, the processors 134_1-134_n may be divided into a first processor group including the processors 134_1-134_m and a second processor group including the processors 134_m+1-134_n. The first processor group may operate to form the B mode image and the second processor group may operate to form the power Doppler mode image. That is, the signal allocating unit 132 may be configured to allocate scan line signals corresponding to the B mode to the first processor group and scan line signals corresponding to the power Doppler mode to the second processor group.

The image synthesizing unit 140 may be configured to synthesize sub-image signals outputted from the first processor group to thereby form a set of ultrasound image signal corresponding to a B mode image frame. Also, the image synthesizing unit 140 may synthesize sub-image signals outputted from the second processor group to thereby form a set of ultrasound image signal corresponding to the power Doppler mode image frame.

Although it is described that the duplex mode includes a B-mode and a PD mode in one embodiment, the diagnostic modes included in the duplex mode are not limited thereto. The duplex mode may include two modes selected from a B mode, a C mode, an M mode, a PW mode, a CW mode, a 3-dimensional mode and the like.

As described above, since the scan line signals are processed by the multi processors or the multi core processor, the performance for processing the scan line signals may be improved. Also, since the hardware-based image processing modules such as the beam forming ASIC, signal processing ASIC, DSP chip, etc. are not required, the costs may be saved and the ultrasound system may be miniaturized- Further since the image processing is carried out by the software, an enhance image may be obtained.

In accordance with one aspect of the present invention, there is provided an ultrasound system, comprising: a probe configured to transmit an ultrasound beam along a plurality of scan lines in a target object and output receive signals based on ultrasound echoes reflected from the target object; a beam forming unit configured to apply delays to the receive signals and sum the delayed receive signals to thereby form scan line signals corresponding to the respective scan lines; a multiprocessing unit including a plurality of processors each being configured to individually process a part of the scan line signals to output sub-image signals, the multiprocessing unit being configured to synthesize the sub-image signals to output ultrasound image signals corresponding to one image frame; and an image processing unit configured to perform image processing upon the ultrasound image signals for display.

In accordance with another aspect of the present invention, there is provided a method of forming ultrasound image signals in an ultrasound system including a plurality of processors, comprising: transmitting an ultrasound beam along a plurality of scan lines in a target object; forming receive signals based on ultrasound echoes reflected from the target object; applying delays to the receive signals and summing the delayed receive signals to thereby form scan line signals corresponding to the respective scan lines; dividing the scan line signals depending on a number of the processors; allocating the divided scan line signals to the processors; individually processing the scan line signals to output sub-image signals; synthesizing the sub-image signals to output an ultrasound image signal corresponding to one image frame; and performing image processing upon the ultrasound image signal for display.

Any reference in this specification to "one embodiment," "an embodiment," "example embodiment," etc means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications arc possible in the component parts and/or arrangement of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:

   a probe for transmitting an ultrasound beam along a plurality of scan lines in a target object and output receive signals based on ultrasound echoes reflected from the target object;
   a beam forming unit for applying delays to the receive signals and sum the delayed receive signals to thereby form scan line signals corresponding to the respective scan lines;
   a multiprocessing unit including a plurality of processors each being configured to individually process a part of the scan line signals to output sub-image signals, the multiprocessing unit being configured to synthesize the sub-image signals to output ultrasound image signals corresponding to one image frame; and
   an image processing unit for performing an image processing upon the ultrasound image signals for display.

2. The ultrasound system of Claim 1, wherein the multiprocessing unit further includes:

   a signal allocating unit for dividing the scan line signals depending on a number of the processors and allocate the divided scan line signals to the processors; and
   a signal synthesizing unit for synthesizing sub-image signals outputted from the processors.

3. The ultrasound system of Claim 2, wherein the multiprocessing unit further includes a memory for storing software algorithm necessary for processing the scan line signals.

4. The ultrasound system of Claim 3, wherein each of the processor is configured to perform amplification, compensation, compression, demodulation and reject upon the scan line signals.

5. The ultrasound system of Claim 4, wherein the signal allocating unit is further configured to divide the processors into a plurality of groups in a duplex mode in which at least two diagnostic modes operate at the same time, and wherein each of the groups processes scan line signals obtained by different diagnostic modes.

6. The ultrasound system of Claim 5, wherein the duplex mode includes at least two diagnostic modes selected from a brightness mode, a color mode, a motion mode, a power Doppler mode and a 3-dimensional mode.

7. The ultrasound system of Claim 4, wherein each of the processors is configured to perform a scan conversion upon the scan line signals.

8. A method of forming ultrasound image signals in an ultrasound system including a plurality of processors, comprising:

   transmitting an ultrasound beam along a plurality of scan lines in a target object;
   forming receive signals based on ultrasound echoes reflected from the target object;
   applying delays to the receive signals and summing the delayed receive signals to thereby form scan line signals corresponding to the respective scan lines;
   dividing the scan line signals depending on a number of the processors;
   allocating the divided scan line signals to the processors;
   individually processing the scan line signals to output sub-image signals;
   synthesizing the sub-image signals to output an ultrasound image signal corresponding to one image frame; and
   performing an image processing upon the ultrasound image signal for display.

9. The method of Claim 8, wherein each of the processors is configured to perform amplification, compensation, compression, demodulation and reject upon the scan line signals

# FIG. 1

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020070140970